# EUROPEAN PATENT SPECIFICATION

(11) **EP 0 263 185 B1**
(45) Date of publication and mention of the grant of the patent: **16.06.1993**
(21) Application number: 87902153.3
(22) Date of filing: 26.03.1987
(51) Int. Cl.: A61L 2/08, A61L 2/10

(54) **SANITARY DEVICE**
SANITÄRANORDNUNG
DISPOSITIF SANITAIRE

(30) Priority: 26.03.1986 JP 67451/86; 26.03.1986 JP 67452/86; 26.03.1986 JP 67453/86; 26.03.1986 JP 67454/86
(43) Date of publication of application: 13.04.1988
(73) Proprietor: HOSHIN KAGAKU SANGYOSHO CO., LTD., Shinjuku-ku, Tokyo 160 (JP)
(72) Inventor: KOJI, Masashi Hoshin Kagaku Sangyosho Co., Ltd., Shinjuku 4-chome Shinjuku-ku Tokyo 160 (JP)
(74) Representative: Schmidt-Evers, Jürgen, Dipl.-Ing.
(86) International application number: PCT/JP87/00187
(87) International publication number: WO 87/05811

(56) References cited:
- WO-A-82/04481
- FR-A- 1 281 437
- GB-A- 1 093 751
- JP-A- 50 685
- US-A- 2 756 470
- US-A- 3 114 038
- US-A- 3 659 096
- US-A- 3 817 703
- US-A- 4 115 280
- US-A- 4 230 947

## Description

The present invention relates to a sanitary device having an element for emitting rays of light which can prevent proliferation of bacteria for an object which is to be maintained in a sanitary state, wherein said element irradiates said object to prevent said bacteria from proliferation.

The present invention relates in particular to such a sanitary device suitable for use with tanks and containers of relatively small size for keeping therein liquids or objects, such as tooth brushes etc., to be maintained in a clean, sanitary condition, and also suitable for use with pipes and nozzles for supplying sterilized water, drinking water or various kinds of drinks which are to be protected from being contaminated by bacteria or the like.

A sanitary device as described above is shown in the US-Patent No. 3,817,703. This known device comprises a laser as the light emitting element. However, lasers are to complicated in construction and to costly for the above indicated use, such as sterilization of tooth brushes in small containers or of liquids flowing out of a faucet.

Ultraviolet lamps are less complicated and less costly than lasers and thus more widely used for sterilization and disinfecting purposes. However, ultraviolet lamps have a lifetime of approximately 2000 to 3000 hours under continuous driving conditions or half of that when used intermittently. Further, due to the size of the glow tubes and stabilizers etc., the ultraviolet lamps are rather large in comparison to small capacity tanks. Thus, it is difficult to make ultraviolet lamps compact in size, which is a requirement for usage with tanks of small capacity. Such tanks are used, for example, for water purifiers and automatic drink vending machines, or as purified water containers, humidifiers, dehumidifiers and air cleaners, and as water containers for holding contact lenses and other objects. Such tanks must be kept clean and in good sanitary condition. In case of utilizing ultraviolet lamps to sterilize the inside of these small tanks, areas which do not have to be sterilized will be irradiated, because it is difficult to minimize the lamp due to the above-mentioned reason. This situation is uneconomical. Further, it is very difficult to mount such ultraviolet lamps in the tank. These disadvantages also occur in the case that the tanks are adapted to the ultraviolet lamps and thus become to large in size as shown in the French Patent No. 1281437 describing a water dispenser for medical use, in which water is disinfected by flowing around and along the whole length of UV-tubes within a tank of corresponding size. Another large-sized tank is shown in the US-Patent No. 3,659,096 describing a fountain, in which UV-lamps are disposed within a cascading curtain of liquid to disinfect the latter.

The US-Patent No. 3,114,038 also discloses a container of relatively large dimensions, in which by means of several UV-lamps air is deodorized or articles like toothbrushes are sterilized.

Further, it is desirable that pipe-shaped portions or nozzles through which sterilized water and drinking water is supplied be maintained in a sanitary, clean condition. Ultraviolet lamps used to sterilize such small, restricted portions cannot be made sufficiently compact in size due to the above-mentioned reason so that they irradiate adjacent areas which need not be irradiated. Such uneconomical usage is shown in the US-Patent No. 2,756,470 that describes a fountain head of a drinking fountain provided with UV-lamps, which are placed at a distance above a discharge nozzle and irradiate same as well as its surroundings with UV-rays. Also, the mounting of such UV-lamps is difficult, as shown in WO-82/04481 describing a faucet which is adapted to accomodate an UV-lamp for sterilizing water passing through and out of the faucet.

Moreover, if ultraviolet lamps are used to sterilize the drink supplying pipe of automatic drink vending machines or the like, it cannot be made compact in size due to the above-mentioned reasons so that it occupies a large space. Also, there may be the risk that the UV-lamp is broken and that harmful insects enter the vending machine.

It is therefore an object of the present invention to provide a sanitary device capable of being utilized in restricted spaces to prevent bacteria from proliferation.

In order to achieve this object the element for emitting rays of light is formed according to the present invention by a light emission diode or by an electro-luminescence element.

Such elements are small in size as compared to the elements used heretofore, and consequently allow to reduce the overall size of the sanitary device, thus permitting its installation in areas of restricted space. Since the output of the light emission diodes or the electro-luminescence elements can be increased by pulse oscillation of large amplitude, a number of such elements may be used and still the size of the sanitary device be kept sufficiently small for the intended application. Further, the light emitting elements according to the present invention not only allow to simplify the construction of the sanitary device, but also have a lifetime of more than 10 times of that of the ultraviolet lamps. On account of their small size, the inventive light emitting elements can be directed to irradiate only those areas that need to be irradiated, thus enhancing the efficiency and economy of use of the sanitary devices, in which they are installed. and thereby protected from harmful influences from the outside.

Preferably, the sanitary device according to the present invention is used with an object having the form of a small tank for containing therein a liquid such as drinking water, drinks and so on to be maintained in a sanitary condition. In this case, the light emission diode or the electro-luminescence element is so disposed as to irradiate the inside of said small tank, whereby bacteria can be prevented from proliferating within said small tank, which in this way is efficiently maintained in a clean and sanitary condition.

The sanitary device according to the invention may also be used with an object having the form of a container for accommodating therein an article to be maintained in a sanitary condition by disposing the light emission diode or the electro-luminescence element so as to irradiate the inside of said container, whereby bacteria can be prevented from proliferating on the article kept in the container.

In case the sanitary device according to the invention is used with an object in form of a nozzle, the light emission diode or the electro-luminescence element is so disposed as to irradiate at least a portion near a tip end of the nozzle to be maintained in a sanitary condition, whereby bacteria can be prevented from proliferating at the mouth of the nozzle. The inventive light emitting elements may be directed so as to irradiate only those areas which need to be exposed to the light rays preventing bacteria profileration. The sanitary device of the present invention as thus enhanced in efficiency and economy of use.

Further, the object to be maintained in a sanitary condition may be a portion of a pipe supplying various kinds of drinks including drinking water, the pipe being made of a material having light transmission property. According to the invention, a plurality of light emission diodes or electro-luminescence elements is disposed around the pipe so as to irradiate the inside of that portion of the drink supplying pipe which has the light transmission property, whereby bacteria can be prevented from proliferating at the portion irradiated by the rays from the light emission diodes or the electro-luminescence elements, these rays including visible light, infrared light and ultraviolet light rays.

The present invention will now be described with reference to the accompanying drawings, in which
Fig. 1 is a cross-sectional view illustrating a first embodiment of the present invention;
Fig. 2 is a cross-sectional view of a main portion illustrating a second embodiment of the present invention;
Fig. 3 is a cross-sectional view illustrating a third embodiment of the present invention;
Fig. 4 is a perspective view illustrating a fourth embodiment of the present invention;
Fig. 5 is a perspective view illustrating a fifth embodiment of the present invention;
Fig. 6 is a perspective view illustrating an example of how to use the embodiment shown in Fig. 5;
Fig. 7 is a diagram and cross-sectional view illustrating a sixth embodiment of the present invention;
Fig. 8 is a cross-sectional view illustrating a seventh embodiment of the present invention;
Fig. 9 is a perspective view illustrating an eighth embodiment of the present invention; and
Fig. 10 is a partly cut-away perspective view illustrating a ninth embodiment of the present invention.

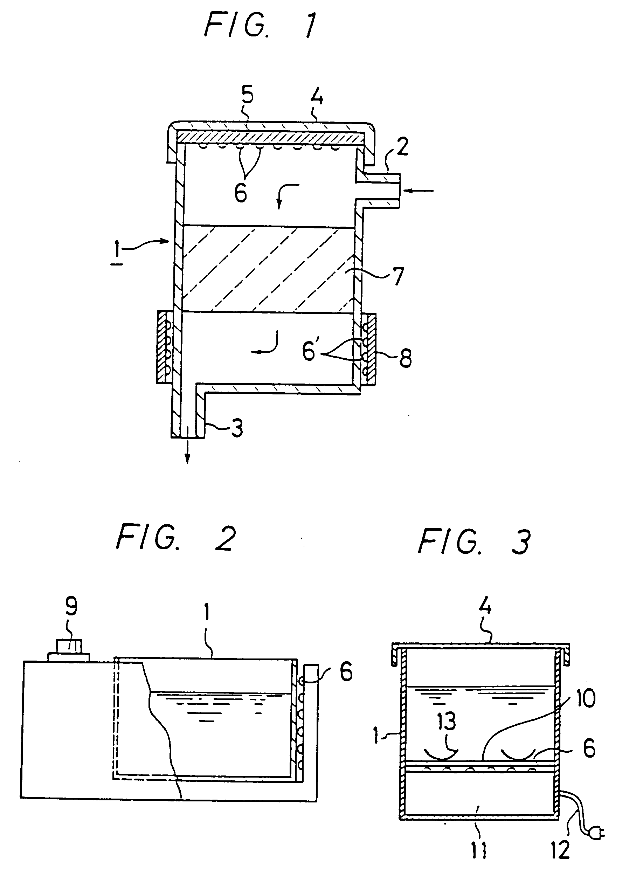

Fig. 1 is a cross-sectional view illustrating the first embodiment in which the present invention is applied to a water purifier. Referring to the figure, a water purifier 1 generally comprises a water entrance 2, a water exit 3, a lid 4, an inner base plate 5, light emission diodes 6 and 6', a filter 7 and an outer base plate 8. Such water purifier 1 can be regarded as a kind of small tank. The outer form of the tank may be either columnar or cubic. The form of the lid 4 may be circular if the outer form of the tank is columnar and may be rectangular if it is cubic. To the rear side of the lid 4, there is fixed the base plate 5 having the light emission diodes 6 mounted thereon. The light emission diodes 6 are arranged to irradiate the water purifier at its portion nearer to the entrance side (primary side) than the filter 7. The outer base plate 8 may be annular if the outer form of the tank is columnar and may be rectangular-annular if it is of a rectangular-ring. The light emission diodes 6' mounted to the inside of the outer base plate 8 irradiate the portion of the tank nearer to the exit side (secondary side) than the filter 7. In this embodiment, the tank must be made of material having light transmission property at least at its portion around which the light emission diodes 6' are located. If there are provided only the light emission diodes 6', it is not necessary to make the tank by the material having light transmission property. The light emission diodes 6, 6' are coated with a proper protective film having light transmission property.

Fig. 2 is a cross-sectional view showing a main portion of the second embodiment in which the present invention is applied to a humidifier. Referring to the figure, this humidifier comprises a water tank 1 for the humidifier, light emission diodes 6 and a volume control 9. Also in this embodiment, the tank 1 must be made of material having light transmission property at its portion opposing to the light emission diodes 6. In this way, the light emission diodes irradiate the inside of the tank from the side surface.

Fig. 3 is a cross-sectional view illustrating the third embodiment of the present invention which is applied to a water receptacle for a contact lens. Referring to the figure, this water receptacle for a contact lens comprises a water receptacle 1, a lid 4, light emission diodes 6, a partition plate 10, a power supply source apparatus 11, a power cord 12 and a contact lens 13. In this embodiment, the inside of the water receptacle 1 is irradiated from its underside so that the partition plate 10 must be made of material having light transmission property. While the power supply source apparatus 11 includes a transformer and a rectifier, the power supply source may be a battery (or a rechargeable battery).

In the first to third embodiments as described above, it may be possible to confirm by providing light receiving elements opposed to the light emission diodes and an indication lamp outside the tank whether or not the light emission diodes are being driven to actually emit rays of light.

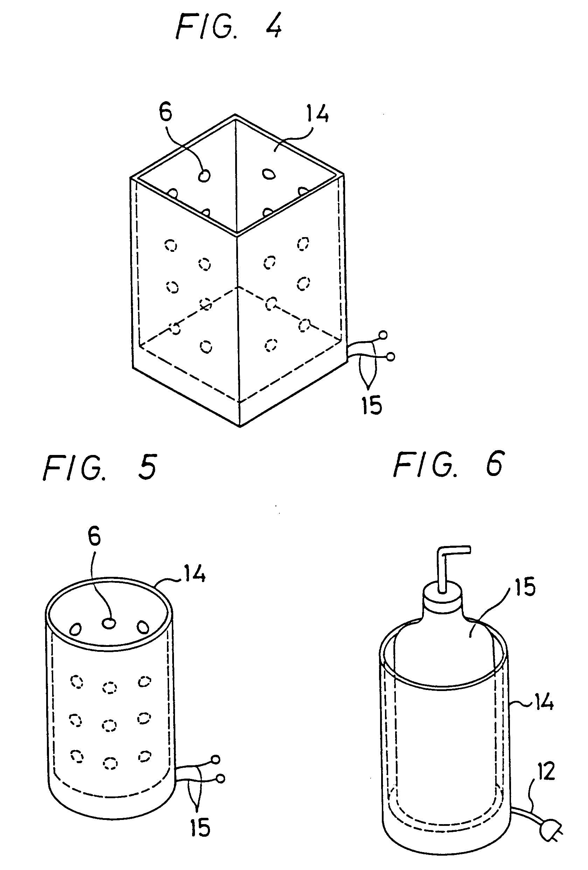

Fig. 4 is a perspective view illustrating the fourth embodiment in which the present invention is applied to a rectangular-shaped container. Referring to the figure, there is provided a container 14. The light emission diodes 6 are arranged on a base plate (not shown) installed on the inner surface of the container 14 and are connected in parallel to a power supply source. The light emission diodes 6 are coated with a proper protective film. Such container 14 may contain therein, in addition to the tooth brushes and the combs, a glass, a cap of microphone and so on which are touched by the user with mouth. If the articles to be contained in this container are made of material having light transmission property, better effects can be achieved. While in the illustrated example the light emission diodes 6 are arranged on the inner surface of the container 14, they may be provided on the bottom surface thereof. While the number of light emission diodes 6 is varied dependent on the output of each diode, the number thereof can be decreased because the output from the light emission diode can be increased by the pulse oscillation having a large amplitude.

Fig. 5 is a perspective view illustrating the fifth embodiment of the present invention which is applied to a cylindrically-shaped container. In the figure, like parts corresponding to those of Fig. 4 are marked with the same references. In the case of the cylindrically-shaped container, the light emission diodes 6 must be located with great care. For example, only the portions thereof to which the light emission diodes 6 are mounted are each made flat or the container 14 is formed as a polygonal column. Other portions are arranged similar to those of Fig. 4.

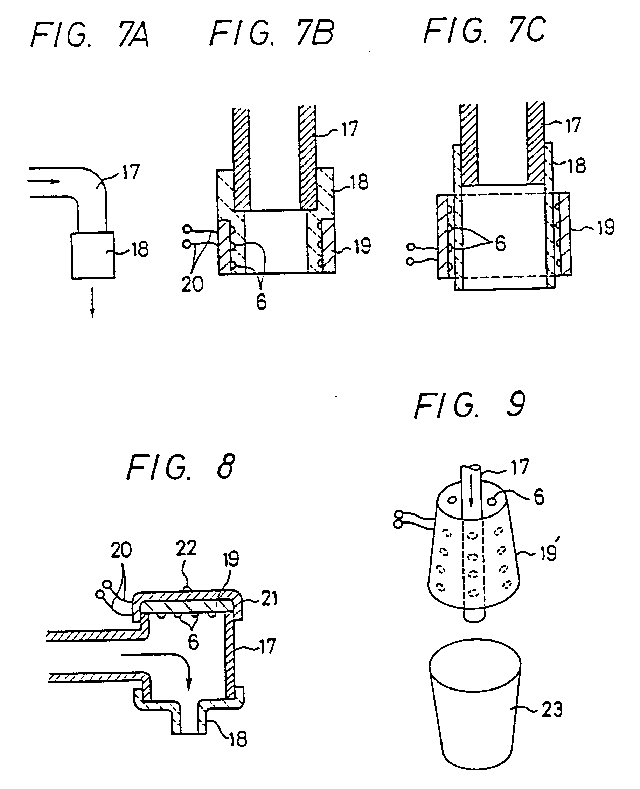

Fig. 6 is a perspective view illustrating an example in which the container of the type shown in Fig. 5 is used actually. The light emission diodes 6 are not shown in Fig. 6. There is provided a bottle of light transmission property which contains purified water. The power supply source may be a commercially-available power supply source or a battery.

While both in the fourth and fifth embodiments the light emission diodes 6 are provided on the inner surface of the container 14, the container 14 may be made of material having light transmission property and the light emission diodes may be arranged around the outer periphery of the container.

Fig. 7 illustrates the sixth embodiment of the present invention, in which Fig. 7A is a diagram showing the appearance thereof, Fig. 7B is a cross-sectional view illustrating the main portion thereof and Fig. 7C is a cross-sectional view illustrating a modified example thereof. Throughout these figures, reference numeral 17 designates a nozzle from which drinking water or germless water is flowed out, 18 a cylindrically-shaped ring engaged with the top of the nozzle, 19 an insulating base plate and 20 lead wires. The cylindrically-shaped ring 18 is made of, for example, material having light transmission property and is formed together with the base plate 19 and the light emission diodes 6. The lead wires 20 are connected to a proper power supply source, and the light emission diodes 6 are coated with a proper protective film. The cylindrically-shaped ring 18 is increased in length so as to form a pipe-shaped one as shown in Fig. 7C, whereby the light emission diodes 6 may be mounted around the outer peripheral surface of such pipe. The ring or pipe 18 attached to the nozzle 17 as above forms a nozzle top portion newly. The number of the light emission diodes 6 may be small if the light emission diode produces a large output, while it is large if the light emission diode produces a small output. The light emission diode 6 can produce a large output by employing an oscillation pulse of large amplitude for intermittently driving the light emission diodes. If the light emission diode is driven by the oscillation pulse, the heat radiation is enhanced to more effectively prevent bacteria from proliferating. If the base plate 19 is formed of a ceramic base plate which is good in heat radiation, the light emission diodes may be driven continuously.

In the embodiment of this type, the light receiving element is provided at the position opposed to the light emission diodes 6 and the outputs therefrom is supplied to an indicator (lamp, etc.) located at the position easy to see the same, thus making it possible to confirm whether the light emission diodes are actually driven to emit rays of light.

Fig. 8 is a cross-sectional view illustrating the seventh embodiment of the present invention. In the figure, like parts corresponding to those of Fig. 7 are marked with the same references. There are shown a cap 21 which is integrally formed together with the base plate 19 and the light emission diodes 6 and a colored light emission diode 22 for confirming the above-mentoned light emission. In this embodiment, the cap 21 is not necessarily made of material having light transmission property, and the nozzle mouth 18 of light transmission property is attached to the top portion of the nozzle 17. This nozzle mouth 18 may be formed as a shower head having a number of small openings.

Fig. 9 is a perspective view illustrating the eighth embodiment of the present invention. Also in this figure, like parts corresponding to those of the sixth embodiment will be identified by the same reference numerals. This embodiment is the application to the nozzle 17 of light transmission property which is long in length. A reversed glass-shaped base plate 19' on the inner side of which the light emission diodes 6 are arranged is mounted to the top portion of the nozzle 17 by proper means. According to this embodiment, not only the exit of the nozzle 17 but also the peripheral portion and the inside of the opening of a glass 23 located above and below the same can be irradiated.

The above-mentioned sixth to eighth embodiments are described as representative examples and various modifications and variations can therefore be effected without departing from the scope of the present invention. For example, the top portion of the nozzle having light transmission property may be formed as a spirally-shaped or zigzag-shaped one and the whole of such portion may be irradiated in the manner same as that shown in Fig. 9. Further, in the embodiment shown in Fig. 7, the mouth of the nozzle top portion 18 is closed and the light emission diodes are mounted on the closed surface so that upon non-use of the nozzle, the light emission diodes are inserted into the top of the nozzle 17.

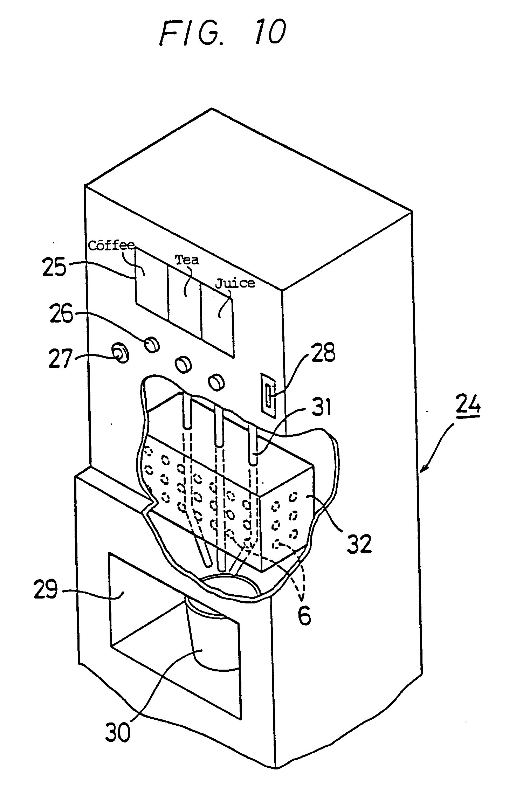

Fig. 10 is a partially cut-away perspective view illustrating the ninth embodiment of the present invention which is applied to an automatic drink vending machine. Referring to the figure, 24 generally shows an automatic drink vending machine, 25 a display panel indicative of the kinds of soft drinks to sell, 26 selection buttons, 27 a confirmation lamp, 28 a slit into which coins are thrown, 29 a large opening from which a desired drink is drawn out, 30 a glass, 31 drink supplying pipes of light transmission property for supplying drinks therethrough and 32 a box-shaped base plate on the inner surface of which the light emission diodes 6 are mounted. In this kind of automatic vending machine, the user picks up a glass from a glass holder, not shown, one by one and puts it in the opening 29. When the user throws coins into the slit 28 and presses the button 26 located under the display of the desired drink, the selected drink of constant quantity contained in each tank, not shown, is supplied through the individual drink supplying pipe 31 and dropped into the glass 30.

The box-shaped base plate 32 is disposed so as to surround the three drink supplying pipes 31 of light transmission property, and the top portion or the bottom portion or both top and bottom portions thereof are made open. Alternatively, the whole thereof may be closed tightly. While the drink supplying pipe 31 of light transmission property is made of, for example, resinous material so as to enable the whole thereof to pass therethrough rays of light, only the portion that should be kept in a sanitary condition is made of material having light transmission property. With this structure, the light emission diodes 6 can irradiate the inside of the drink supplying pipe. Further, they can irradiate the portion near the mouth of the pipe if the bottom of the box-shaped base plate 32 is opened, thus suppressing bacteria from proliferating at the portion irradiated by rays of light.

In this embodiment, the light receiving elements are disposed within the box-shaped base plate 32 to receive rays of light emitted from the light emission diodes 6, and also connected to the confirmation lamp 27 installed on the front panel of the automatic vending machine 24, thus making it possible to confirm from the outside whether or not the light emission diodes 6 are actually being operated to emit rays of light. This arrangement, however, may be omitted. While in the illustrated embodiment the base plate 32 to which the light emission diodes 6 are mounted is formed as a boxed-shaped one, it may be cylindrically-shaped base plate. Further, such a version is also possible that when the user holds the glass 30, the light emission diodes are disabled so as to protect the user's hands from the irradiation of light.

The present invention is not limited to the above-mentioned embodiments, but can be widely applied to various tanks such as tanks of small capacity used in automatic drink vending machines, containers for purified water, supplying tanks for flush toilets and water tanks for flush toilets of washing douches and drier type; and these small tanks can be efficiently maintained in a clean and sanitary condition.

The invention can be embodied in a version in which metals or non-metals producing electrons by irradiation of rays of light are provided on the surface which is irradiated by rays of light, whereby photoelectrons are produced to prevent the proliferation of bacteria more efficiently. Also, ion action is caused by the electrons and this ion action is utilized to purify or deodorize air, water or the like.

## Claims

1. A sanitary device having an element for emitting rays of light which can prevent proliferation of bacteria for an object which is to be maintained in a sanitary state, wherein said element irradiates said object to prevent said bacteria from proliferation, **characterized** in that said element for emitting rays of light is formed by a light emission diode or by an electro-luminescence element.

2. A sanitary device according to claim 1, in which said object is a small tank for containing therein liquid such as drinking water, drinks and so on to be maintained in a sanitary condition, **characterized** in that said light emission diode or said electro-luminescence element, respectively, is so disposed as to irradiate the inside of said small tank, whereby bacteria can be prevented from proliferating within said small tank

3. A sanitary device according to claim 1, in which said object is a container for accommodating therein an article to be maintained in a sanitary condition, **characterized** in that said light emission diode or said electroluminescence element, respectively, is so disposed as to irradiate the inside of said container, whereby bacteria can be prevented from proliferating in said article kept in said container.

4. A sanitary device according to claim 1, in which said object is a nozzle, **characterized** in that said light emission diode or said electro-luminescence element, respectively, is so disposed as to irradiate at least a portion near a tip end of said nozzle to be maintained in a sanitary condition, whereby bacteria can be prevented from proliferating at the mouth of said nozzle.

5. A sanitary device according to claim 1, in which said object is a portion of a drink supplying pipe which at least to be maintained in a sanitary condition and is made of material having light transmission property, **characterized** in that said light emission diode or said electro-luminescence element, respectively, is disposed in plurality around said pipe so as to irradiate the inside of said light transmission property portion of said drink supplying pipe, whereby bacteria can be prevented from proliferating at said portion irradiated by said rays from said light emission diode or said electro-luminescence element.

## Patentansprüche

1. Hygienevorrichtung mit einem Element zur Emmission von Lichtstrahlen, das bei einem Gegenstand, der in einem hygienisch einwandfreien Zustand gehalten werden soll, ein Bakterienwachstum verhindern kann, wobei dieses Element diesen Gegenstand bestrahlt, um zu verhindern, daß diese Bakterien sich vermehren,
dadurch gekennzeichnet,
daß dieses Element zur Emmission von Lichtstrahlen von einer LED-Diode oder einem Elektrolumineszenz-Element gebildet wird.

2. Hygienevorrichtung nach Anspruch 1, bei der dieser Gegenstand ein kleiner Behälter für Flüssigkeiten wie Trinkwasser, Getränke etc. ist, die in einem hygienisch einwandfreien Zustand gehalten weren sollen, dadurch gekennzeichnet, daß diese LED-Diode bzw. dieses Elektrolumineszenz-Element dergestalt angeordnet ist, daß das Innere dieses kleinen Behälters bestrahlt wird, wodurch sich verhindern läßt, daß sich in diesem kleinen Behälter Bakterien vermehren.

3. Hygienevorrichtung nach Anspruch 1, bei der dieser Gegenstand ein Behälter für einen Artikel ist, der in einem hygienisch einwandfreien Zustand gehalten weren soll, dadurch gekennzeichnet, daß diese LED-Diode bzw. dieses Elektrolumineszenz-Element dergestalt angeordnet ist, daß das Innere dieses Behälters bestrahlt wird, wodurch sich verhindern läßt, daß sich auf diesem in dem Behälter aufbewahrten Artikel Bakterien vermehren.

4. Hygienevorrichtung nach Anspruch 1, bei der dieser Gegenstand eine Düse ist, dadurch gekennzeichnet, daß diese LED-Diode bzw. dieses Elektrolumineszenz-Element dergestalt angeordnet ist, daß mindestens ein Abschnitt in der Nähe der Spitze dieser Düse, die in einem hygienisch einwandfreien Zustand gehalten weren soll, bestrahlt wird, wodurch sich verhindern läßt, daß sich am Mund dieser Düse Bakterien vermehren.

5. Hygienevorrichtung nach Anspruch 1, bei der dieser Gegenstand wenigstens ein Abschnitt einer Getränkezapfleitung ist, der in einem hygienisch einwandfreien Zustand gehalten werden soll und aus einem lichtdurchlässigen Werkstoff hergestellt ist, dadurch gekennzeichnet, daß mehrere dieser LED-Dioden bzw. dieser Elektrolumineszenz-Elemente dergestalt um diese Leitung herum angeordnet sind, daß das Innere dieses lichtdurchlässigen Abschnitts dieser Getränkezapfleitung bestrahlt wird, wodurch sich verhindern läßt, daß sich an diesem Abschnitt, der durch die Strahlen dieser LED-Dioden bzw. dieser Elektrolumineszenz-Elemente bestrahlt wird, Bakterien vermehren.

## Revendications

1. Appareil hygiénique, comportant un élément destiné à émettre des rayons lumineux qui peuvent empêcher la prolifération des bactéries sur un objet qui doit être maintenu à un état hygiénique, dans lequel l'élément irradie l'objet afin qu'il empêche la prolifération des bactéries, caractérisé en ce que l'élément destiné à émettre des rayons lumineux est formé d'une diode photoémissive ou d'un élément électro-luminescent.

2. Appareil hygiénique selon la revendication 1, dans lequel l'objet est un petit réservoir destiné à contenir un liquide tel que de l'eau de boisson, une boisson, etc. afin qu'il reste à un état hygiénique, caractérisé en ce que la diode photoémissive ou l'élément électro-luminescent respectivement a une position telle qu'il irradie l'intérieur du petit récipient, si bien que les bactéries ne peuvent pas proliférer à l'intérieur du petit récipient.

3. Appareil hygiénique selon la revendication 1, dans lequel l'objet est un réceptacle destiné à contenir un article qui doit être conservé à un état hygiénique, caractérisé en ce que la diode photoémissive ou l'élément électro-luminescent respectivement a une position telle qu'il irradie l'intérieur du réceptacle, si bien que les bactéries ne peuvent pas proliférer dans l'article conservé dans le réceptacle.

4. Appareil hygiénique selon la revendication 1, dans lequel l'objet est une buse, caractérisé en ce que la diode photoémissive ou l'élément électro-luminescent respectivement a une position telle qu'il irradie au moins une partie proche de l'extrémité du bout de la buse qui doit être maintenue à un état hygiénique, si bien que les bactéries ne peuvent proliférer à l'embouchure de la buse.

5. Appareil hygiénique selon la revendication 1, dans lequel l'objet est une partie d'un tube de distribution de boisson qui doit être maintenu au moins à un état hygiénique et qui est formé d'un matériau ayant des propriétés de transmission de la lumière, caractérisé en ce que plusieurs diodes photoémissives ou éléments électro-luminescents respectivement sont placés autour du tube afin qu'ils irradient l'intérieur de la partie de transmission de lumière du tube de distribution de boisson, si bien que les bactéries ne peuvent pas proliférer sur la partie irradiée par les rayons de chaque diode photoémissive ou élément électro-luminescent.
